Europäisches Patentamt

European Patent Office

Office européen des brevets

(11) Veröffentlichungsnummer: **0 541 546 B1**

## EUROPÄISCHE PATENTSCHRIFT

(45) Veröffentlichungstag der Patentschrift: **05.10.94**

(21) Anmeldenummer: **90916242.2**

(22) Anmeldetag: **12.11.90**

(86) Internationale Anmeldenummer:
**PCT/DE90/00875**

(87) Internationale Veröffentlichungsnummer:
**WO 91/07357 (30.05.91 91/12)**

(51) Int. Cl.5: **C03C 3/16**, C03C 10/02,
A61L 27/00

(54) GLASIGES ODER GLASIG-KRISTALLINES MATERIAL MIT SCHNELLER LÖSLICHKEIT UND VERFAHREN ZU SEINER HERSTELLUNG.

(30) Priorität: **13.11.89 DD 334524**

(43) Veröffentlichungstag der Anmeldung:
**19.05.93 Patentblatt 93/20**

(45) Bekanntmachung des Hinweises auf die
Patenterteilung:
**05.10.94 Patentblatt 94/40**

(84) Benannte Vertragsstaaten:
**AT CH DE ES FR GB IT LI SE**

(56) Entgegenhaltungen:
**DE-A- 3 142 813**
**GB-A- 2 031 867**

(73) Patentinhaber: **BIOVISION GmbH Entwicklung,**
**Herstellung und Vertrieb von Biomaterialien**
**Am Vogelherd 1**
**D-98693 Ilmenau (DE)**

(72) Erfinder: **BERGER, Georg**
**Rosenthaler Strasse 5**
**D-1113 Berlin (DE)**

Erfinder: **SAUER, Renate**
**Amalienstrasse 24**
**D-1120 Berlin (DE)**
Erfinder: **STEINBORN, Gabriele**
**Pichelsberger Str. 3**
**D-1100 Berlin (DE)**
Erfinder: **KNÖFLER, Wolfram**
**Kantstr. 34**
**D-7030 Leipzig (DE)**
Erfinder: **GRAF, Hans-Ludwig**
**Volkgartenstr. 26/56**
**D-7024 Leipzig (DE)**
Erfinder: **THIEME, Volker**
**Otto-Militzer-Str. 17**
**D-6902 Jena (DE)**
Erfinder: **DRESSEL, Horst**
**Wilhelm-Pieck-Str. 20**
**D-6325 Ilmenau (DE)**
Erfinder: **GÜNTHER, Olaf**
**Bertold-Brecht-Str. 16**
**D-6325 Ilmenau (DE)**

EP 0 541 546 B1

(74) Vertreter: **Liedtke, Klaus, Dr. et al**
**Cyriakstrasse 27**
**Postfach 956**
**D-99019 Erfurt (DE)**

**Beschreibung**

Anwendungsgebiet der Erfindung

Die Erfindung betrifft Herstellungsverfahren von Materialien, die definiert schnell löslich sind und in Bezug auf ihre Anwendung keine toxischen Substanzen freisetzen. Sie können überall dort eingesetzt werden, wo definierte Lösungsgeschwindigkeiten in einem weiten Bereich notwendig sind, wie bei der Applikation als Knochensubstitut, Bindegewebesubstitut, bei der Bindegewebeinduktion, als Trägermaterial für anorganische Komponenten, wie z.B. Spurenelemente, bei Futterzusatz- bzw. Düngemitteln.

Charakteristik der bekannten technischen Lösungen

Materialien mit extrem schneller Löslichkeit sind an sich bekannt, wenn man natürlich vorkommende bzw. synthetisch erzeugte Salze in die Betrachtung einschließt. Dies gilt sowohl für den Bereich der Dünge- und Futtermittel etc. als auch für das Anwendungsgebiet des Knochenersatzes. Jedoch wird durch die extrem schnelle Freisetzung aller Bestandteile häufig eine unphysiologische, ja teilweise toxische Konzentration ("Überdüngung") von an sich physiologischen Bestandteilen dadurch erzeugt. Ein weiterer Nachteil ist, daß demzufolge von diesen Stoffen keine Depotwirkung ausgeht. Dies gilt auch für die üblicherweise verwendeten Phosphat-, Superphosphat- und Triplesuperphosphat-Düngemittel. In der Knochen- bzw. Bindegewebesubstitution haben sich derart einfache Salze ebenfalls nicht bewährt. Um diesen Nachteil im angesprochenen medizinischen Fachbereich zu beheben, wurden sogenannte resorbierbare Knochenersatzmaterialien auf der Basis von Tricalciumphosphat oder Hydroxylapatit eingeführt, deren Resorptionsdauer in sehr vielen Anwendungsfällen nun wiederum zu lang ist.

Technische Lösungen für den Düngemittelbereich, die eine Depotwirkung für diese Anwendung einschließen, gründen sich auf Materialien, die sehr hohe Phosphatgehalte aufweisen und als Metaphosphat-Gläser einzuordnen sind (vergl. hierzu: KNOTT, P.,Glasses - Agricultural applications, Glastechn. Ber. 62 (1989)[1], 29-34; GB-P 2099702 A). Dies gilt ebenso für die Futtermittel, speziell jedoch für Materialien, die subkutan implantiert in der Tierproduktion angewendet werden können, obwohl in diesem Fall die löslichen Stoffe vorzugsweise als Trägermaterialien dienen, um Mangelerscheinungen auszugleichen, und zwar besonders an Magnesium, Kupfer, Kobalt und/oder Selen.

Auch hier - wie im Falle der Düngemittel - ist der hohe Phosphatgehalt, bedingt durch die Wahl von Metaphosphat-Gläsern nachteilig (vergl. hierzu: HARENZ, H., Phosphoreinsatz und Phosphorverluste im Bereich Landwirtschaft - Ernährung - Haushalt der DDR aus ökonomischer und ökologischer Sicht, Aus Arb. Plenum Kl. AdW DDR.-Berlin 14(1989)3., 25-43).

Auf dem Gebiet der bioaktiven Knochenersatzmaterialien werden, wie bereits erwähnt, resorbierbare Materialien nur aus dem Bereich der Calciumorthophosphate eingesetzt, wobei deren ursprünglich geringe Löslichkeit durch verschiedene chemische und mechanische Verfahren innerhalb bestimmter Grenzen variiert werden kann. (Vergl. hierzu auch: Bauer, G.; Hohenberger, G.: Ursachen des unterschiedlichen Verhaltens von bioaktiven Calciumphosphat-Keramiken im Organismus, cfi/Ber. DKG 66(1989)[1/2], 23 - 27.)

Im EP 0237043 wird die Darstellung von calciumphosphathaltigen biokompatiblen Schichtkörpern beschrieben, wobei der Grundkörper eine Schicht aufweist, die eine höhere Calciumfreisetzung ermöglicht als der Grundkörper selbst. Andere Lösungen verweisen ausdrücklich auf die Ionen-Donator-Wirkung sowohl von Calciumionen als auch von Magnesium-Natrium- und Kaliumionen. In DE 2346739 wird unter diesem Gesichtspunkt ein Sinterprodukt aus Apatit, Glas/Vitrokeram und Permutit zur Verwendung als Implantatmaterial beschrieben.

Die in DD 248351 bzw. DE-05 3306648 beschriebenen bioaktiven Materialien vom $CaO$-$P_2O_5$-$SiO_2$-Typ zeigen in granulierter Form eine stärkere Freisetzung von Calcium-, Natrium-, Kalium- und Magnesiumionen, jedoch werden dabei gleichzeitig auch größere Mengenanteile an Silicium freigesetzt, so daß eine Verwendung dieser Materialien nur in kompakter Form möglich ist.

Bisher nicht entwickelt wurde ein Material mit definiert schneller Löslichkeit, das gerade jenen Bereich abdeckt, der auf der einen Seite eine Depotwirkung bei Düngemitteln und bei o.g. subkutaner Implantation bei der Tierproduktion garantiert und auf der anderen Seite die (noch) zu geringe Resorptionsfähigkeit bei Knochenersatzmaterialien überwinden hilft. Dieses Material sollte aus der Sicht der Düngemittel wie auch ebenso der Knochenersatzmaterialien etc. nicht zu hohe Phosphorgehalte aufweisen, wie es den Metaphosphat-Gläsern eigen ist. Dennoch sollte das Material gut sinterbar, besser noch schmelzbar und gießbar sein, was einen weiteren Vorteil insbesondere hinsichtlich der Verarbeitbarkeit und der Homogenität des Produktes darstellt.

Es ist das Ziel der Erfindung, die geschilderten Nachteile des Standes der Technik zu überwinden.

Wesen der Erfindung

Der Erfindung liegt die Aufgabe zugrunde, ein Material bereitzustellen, das sowohl in als auch ex vivo definiert schnell löslich ist und sich gut sintern oder schmelzen, vergießen und in Granulatform herstellen läßt.

Erfindungsgemäß wurde ein Material mit diesen Merkmalen entwickelt, das darüber hinaus noch weitere vorteilhafte Eigenschaften aufweist, die weiter unten näher beschrieben werden.

Schmilzt man Gemenge der Zusammensetzung von (Angaben in Masseteile in %):

22 - 45 CaO
8 - 20 $Na_2O$
O - 14 $K_2O$, vorzugsweise 0,1 - 14;
0 - 15 MgO, vorzugsweise 0,1 - 15;
30 - 55 $P_2O_5$
0 - 15 $SiO_2$, vorzugsweise 0,1 - 15;
0 - 40 $Na_2SO_4$ und/oder $K_2SO_4$, vorzugsweise 0,1 - 35

ein, vergießt oder frittet sie, so erhält man spontan kristallisierte Glaskeramiken, die überraschenderweise eine bislang in der ASTM-Kartei sowie in der einschlägigen Fachliteratur nicht ausgewiesene kristalline Phase enthalten, die im Rahmen dieser Beschreibung mit "X" bezeichnet wird, bzw. man erhält Mischkristalle dieser Phase "X".

Röntgendiffraktometrisch wird diese Phase "X" bzw. werden Mischkristalle von dieser Phase in etwa durch folgende d-Werte und Intensitäten charakterisiert:

Zusammensetzungsbeispiel c
d-Wert: 3,945 3,650 3,384 3,199 2,885 2,717 2,552 2,351 2,239 2,164 1,980 1,827 1,597 1,569 1,517
Intensitäten: 20 20 2 8 90 100 10 10 20 8 40 8 10 10 10

Zusammensetzungsbeispiel a
d-Wert: 3,904 3,618 3,347 3,189 2,851 2,679 2,529 2,321 2,209 2,141 1,953 1,808 1,578 1,547 1,498
Intensitäten: 20 20 2 8 90 100 10 10 20 8 40 8 10 10 10

Zusammensetzungsbeispiel d
d-Wert: 3,892 3,611 3,338 3,15 2,844 2,667 2,523 2,310 2,199 2,135 1,945 1,804 1,571 1,539 1,495
Intensitäten: 15 20 2 8 90 100 10 10 20 8 40 8 10 10 10

Zusammensetzungsbeispiel b
d-Wert: 3,875 3,600 3,325 3,12 2,835 2,663 2,514 2,303 2,195 2,131 1,941 1,800 1,569 1,537 1,491
Intensitäten: 20 20 2 8 90 100 10 10 20 9 40 8 10 8 8

Diese kristalline Phase ist damit der in der Literatur als Phase "A" bezeichneten Phase ähnlich (vergl. hierzu: Ando, J.; Matsuno, S.: $Ca_3(PO_4)_2$-$CaNaPO_4$ System, Bulletin Chem. Soc. Japan 41(1968) 342 - 347), von der sie sich jedoch durch erhebliche Linienverschiebungen und Intensitätsänderungen sowie durch das Fehlen einer starken Beugungslinie an der Netzebene (421) unterscheidet. Die Phase "A" wurde von Ando als eine Überstruktur des hexagonalen Calciumnatriumorthophosphats beschrieben, wobei er sowohl die Hoch- als auch die Tieftemperaturform des $CaNaPO_4$ als alpha- bzw. beta-Rhenanit bezeichnet und die bloße Formulierung Rhenanit folglich beide Formen einschließt. Diesem Sprachgebrauch bzw. dieser Definition des Rhenanits schließen wir uns in der vorliegenden Beschreibung an.

Dem Strukturtyp von "A" ist auch die Phase "X" zu zuordnen, wobei allem Anschein nach bis über die Hälfte des Natriums durch Kalium ersetzt werden kann und auch Calcium teilweise durch Magnesium in dieser Struktur substituiert werden kann. Diese Substitutionen werden im allgemeinen, so auch hier, als Mischkristallbildung bezeichnet.

Die diese Phase enthaltenden Materialien zeigen nun auch die gewünschten Eigenschaften hinsichtlich einer definiert schnellen Löslichkeit, insbesondere im Hinblick auf die Knochensubstitution. In tierexperimentellen Studien wurde zudem überraschenderweise gefunden, daß ein bestimmter Teil dieser Stoffe wiederum die Bindegewebsbildung induziert.

4

EP 0 541 546 B1

Die neuen Materialien liegen im abgekühlten Zustand bei Raumtemperatur als Glas oder glaskristallines Material vor, können jedoch prinzipiell in den glaskristallinen Zustand überführt werden und weisen im wesentlichen an sich physiologische Bestandteile auf. Die Lösungsgeschwindigkeit der Materialien wird entsprechend der Anwendung in weiten Grenzen, wie weiter unten genau beschrieben, so eingestellt, daß keine toxischen Reaktionen bzw. Überkonzentrationen von jedweden Bestandteilen impliziert werden.

Eine Aufweitung des Schmelzbereiches führt über die Phase "X" hinaus zu weiteren, an sich bekannten kristallinen Phasen. Es können zusätzlich oder jeweils für sich allein die Phase "A" bzw. deren Mischkristalle, Rhenanit bzw. dessen Mischkristalle in dem erfindungsgemäß erzeugten Material vorhanden sein. Diese Zusammensetzungsvariationen eignen sich ebenso zur Applikation in den genannten Anwendungsgebieten im Sinne der Zielstellung, wie auch die Einbeziehung der an sich bekannten Isomorphiebeziehungen der Sulfate des Kaliums und Natriums.

Dieses erweiterte Zusammensetzungsgebiet erstreckt sich damit auf die bereits eingangs genannten Komponenten in ihren Masseanteilen in %:

20 - 55 CaO

5 - 25 $Na_2O$

0,01-15 $K_2O$

0 - 15 MgO

30 - 50 $P_2O_5$

0 - 15 $SiO_2$

0 - 40 $Na_2SO_4$ und/oder $K_2SO_4$

Eine weitere Erhöhung des Calciumorthophosphatanteils führt jedoch zu zunehmend schwerer bzw. nicht schmelzbaren und nicht gießbaren Materialien, die sich damit sowohl von ihrem Herstellungsverfahren als auch in ihren Eigenschaften (Löslichkeiten) den bekannten Materialien nähern bzw. sich von den Erfindungszielen der vorliegenden Schrift entfernen.

Besonders vorteilhafte Ausführungsformen liegen daher in den Konzentrationsbereichen (Masseanteile in %)

21 - 50 CaO, insbesondere 23 - 50

5 - 20 $Na_2O$, insbesondere 6 - 20

0,1 - 14 $K_2O$, insbesondere 2 - 14

0,1- 12 MgO, insbesondere 0,5- 10

32 - 48 $P_2O_5$, insbesondere 35- 48

0,1- 15 $SiO_2$, insbesondere 1 - 10

0,1- 35 $Na_2SO_4$ und/oder $K_2SO_4$, insbesondere 0,1 - 20

Weitere bevorzugte, erfindungsgemäße Ausführungsformen sind:

22 - 55 CaO, 6 - 12 $Na_2O$, 3 - 14 $K_2O$, 2 - 8 MgO, 37 - 43 $P_2O_5$, 0,5 - 10 $SiO_2$, 0,5 - 20 $Na_2SO_4$ und/oder $K_2SO_4$.

Das erfindungsgemäße Material ist weiterhin dadurch gekennzeichnet, daß es sich leicht sintern läßt bzw. bei einer Temperatur im Bereich von 1200 bis 1550 °C in eine gießfähige Schmelze überführt werden kann. Weitere Kennzeichen des Materials bestehen darin, daß im Falle der Schmelzbildung die Schmelze bei einer Abkühlgeschwindigkeit von größer als etwa 150 °C pro Minute in den glasigen Zustand bei Raumtemperatur überführt werden kann und daß die Schmelze bei Abkühlung unter normaler Abkühlgeschwindigkeit bzw. unter einer Abkühlgeschwindigkeit, die langsamer als etwa 35 °C pro Minute erfolgt, spontan kristallisiert. Dabei bildet sich ein feinkristallines Gefüge aus.

Desweiteren wurde gefunden, daß spontan kristallisiertes Material gleicher chemischer Zusammensetzung wie das (unter extremen Abkühlbedingungen erhaltene) entsprechende Glas oder das aus dem Glas durch Temperung erzeugtes kristallines Material jeweils unterschiedliche Löslichkeiten aufweisen.

Es wurde weiterhin gefunden, daß die spontan kristallisierte Glaskeramik als kristalline Hauptbestandteile mindestens eine der Phasen des Rhenanits, Mischkristalle des Rhenanits, die Phase "A", Mischkristalle der Phase "A", die bereits oben genannte neue Phase "X" und/oder Mischkristalle der Phase "X" enthält. Das Material kann zusätzlich noch Glaserit und/oder kristallines Kaliumsulfat enthalten.

Wenn das Material als kristalline Hauptbestandteile Rhenanit bzw. Mischkristalle des Rhenanits enthält, so liegt die Zusammensetzung des Materials im Gemenge wie folgt vor (in Masseanteile in % und auf Oxidbasis berechnet):

30 -40 CaO; 15 - 20 $Na_2O$; 0 - 1 $K_2O$, vorzugsweise 0,01 - 0,1 $K_2O$; 0 - 5 MgO, vorzugsweise 0,1 - 5 MgO; 40 - 55 $P_2O_5$; 0 - 15 $SiO_2$, vorzugsweise 0,1 - 8 $SiO_2$; 0 - 30 $Na_2SO_4$ und/oder $K_2SO_4$, vorzugsweise 0,1 - 25 $Na_2SO_4$ und/oder $K_2SO_4$.

Unter "kristalliner Hauptbestandteil" wird verstanden, daß der prozentuale Anteil der Komponente höher liegt als der Anteil anderer im Material vorhandener Komponenten.

5

Wenn das Material als kristalline Hauptbestandteile die Phase "A" enthält, so liegt die Zusammensetzung des Materials im Gemenge wie folgt vor (in Masseanteile in % und auf Oxidbasis berechnet): 40 - 50 CaO; 8 - 20 $Na_2O$; 0 - 1 $K_2O$, vorzugsweise 0,1 - 1 $K_2O$; 0 - 5 MgO, vorzugsweise 0,1 - 5 MgO; 40 - 50 $P_2O_5$; 3 - 20 $SiO_2$; 0 - 30 $Na_2SO_4$ und/oder $K_2SO_4$, vorzugsweise 0,1 - 25 $Na_2SO_4$ und/oder $K_2SO_4$.

Wenn das Material als kristalline Hauptbestandteile die Phase "X" bzw Mischkristalle der Phase "X" enthält, so liegt die Zusammensetzung des Materials im Gemenge wie folgt vor (in Masseanteile in % und auf Oxidbasis berechnet): 22 - 45 CaO; 8 - 20 $Na_2O$; 0 - 14 $K_2O$, vorzugsweise 0,1 - 14 $K_2O$; 0 - 15 MgO, vorzugsweise 0,1 - 15 MgO; 30 - 55 $P_2O_5$; 0 - 15 $SiO_2$, vorzugsweise 0,1 -15 $SiO_2$; 0 - 40 $Na_2SO_4$ und/oder $K_2SO_4$, vorzugsweise 0,1 - 35 $Na_2SO_4$ und/oder $K_2SO_4$.

Ein besonderer Vorteil der Erfindung ist es, daß das Material biokompatibel ist, teilweise sogar bioaktiv im Sinne einer direkten Knochenanlagerung oder unter ständigem Lösen die Bildung neuen Knochengewebes ermöglicht. Es fördert andererseits auch die Bindegewebsbildung. Das Material kann beispielsweise als Granulat eingesetzt werden mit einer Körnung im bereich von 63 - 500 $\mu$m.

Gegenstand der Erfindung ist weiterhin ein Verfahren zur Herstellung eines glasigen oder glasigkristallinen Materials mit schneller Löslichkeit, das darin besteht, daß man ein Gemenge, bestehend aus (in Masseanteile in % und auf Oxidbasis berechnet): 20 - 55 CaO; 5 - 25 $Na_2O$; 0 - 15 $K_2O$; 0 - 15 MgO; 30 - 55 $P_2O_5$; 0 - 15 $SiO_2$; 0 - 40 $Na_2SO_4$ und/oder $K_2SO_4$ mindestens 10 Minuten lang bei einer Temperatur von etwa 1200 bis 1580 °C sintert oder schmilzt und die Schmelze abkühlt. Bevorzugt einsetzbar sind die bereits weiter oben genannten bevorzugten Zusammensetzungen.

Wie bereits weiter oben dargestellt, kann die Abkühlung mit einer sehr hohen Abkühlgeschwindigkeit von wenigstens 150, besser jedoch $5*10^2$ °C pro Minute erfolgen und dabei ein glasiges Material erhalten werden.

Doch der Hauptweg der Herstellung von erfindungsgemäßen schnell löslichen Materialien besteht im Schmelzen mit nachfolgender spontaner Kristallisation. Daher sollen im weiteren alle Ausführungen vorzugsweise auf diesen Verfahrensweg ausgerichtet sein. Dabei wird die Schmelze mit einer Geschwindigkeit von kleiner als ca. 35 °C pro Minute abgekühlt, wobei die spontane Kristallisation auftritt.

Vorteilhaft wird das spontan kristallisierte Material einem üblichen Temperungsprozeß unterzogen. Das erfolgt im Temperaturbereich von ca. 600 bis 1200 °C, in Abhängigkeit von der chemischen Zusammensetzung und der zu erzeugenden Kristallphase, um den kristallinen Anteil - in der Regel handelt es sich dabei um die kristalline Hauptphase - noch weiter zu erhöhen. Der Zusammenhang von chemischer Zusammensetzung und Kristallphase wurde bereits weiter oben generell dargestellt. Es treten durch die erfindungsgemäße Behandlung die Kristallphasen des Rhenanits, der Phase "A", Mischkristalle der Phase "A", der Phase "X", Mischkristalle der Phase "X", Glaserit und/oder kristallines Kaliumsulfat in Erscheinung.

Für das erfindungsgemäße Verfahren vorteilhaft ist es, $P_2O_5$ in Form von Phosphorsäure einzusetzen.

Eine vorteilhafte Materialform ist die Granulatform, so daß das aus der Schmelze abgekühlte und gegebenenfalls getemperte Material mittels üblicher Verfahren zerkleinert und klassiert wird.

Wenn der Gehalt an $Na_2SO_4$ und/oder $K_2SO_4$ etwa gleich oder größer als 3 Masseanteile in % im Ausgangsgemenge beträgt, ist es vorteilhaft, das Granulat einer Behandlung mit destilliertem Wasser über einen Zeitraum von 0,1 bis 10 Stunden bei erhöhter Temperatur, vorzugsweise bei ca. 80 bis 100 °C auszusetzen. Dadurch wird eine Erhöhung der inneren Oberfläche des Materials erreicht, was sich in einer Erhöhung der Löslichkeit ausdrückt, und der Sulfatanteil kann, - wenn er nicht erwünscht ist -, reduziert werden. Darüber hinaus kann das auf diese Weise zurückgewonnene Sulfat dem Verarbeitungsprozeß wieder zugeführt werden.

Wie bereits ausgeführt, kann das neue schnell lösliche Material als resorbierbares Knochenersatzmaterial eingesetzt werden oder auch zur Bindegewebsinduktion.

Hierbei ist es zweckmäßig, Granulat in gewünschter Körnung in die entsprechenden Körperregionen, z.B. postoperative Hohlräume im menschlichen oder tierischen Körper, einzubringen, wobei dies auch im Gemisch mit anderen Stoffen erfolgen kann, z.B. mit weniger schnell löslichen biokompatiblen Materialien gem. DD 258713, Trägerfolien usw. Beim Einsatz zur Bindegewebsinduktion entscheidet ebenfalls zunächst die konkrete gewählte Zusammensetzung über den Erfolg der Behandlung; es können jedoch auch Gemische mit anderen Stoffen, die bekanntermaßen zu keiner Knochenanlagerung führen, in diesem Fall zur Anwendung gebracht werden.

Das Material kann auch zur Beschichtung von Implantatformkörpern für den Hartgewebeersatz, bestehend aus Metallen oder Legierungen und/oder Keramiken, vorzugsweise $Al_2O_3$-Keramiken eingesetzt werden. damit würde es zu einer verbesserten und vor allem schnelleren Einheilungsphase beitragen.

Es wurde weiterhin gefunden, daß die erfindungsgemäßen Materialien mit schneller Löslichkeit in der eingangs beschriebenen Zusammensetzung einschließlich der vorteilhaften Zusammensertzungsbereiche besonders erfolgreich als Düngemittel eingesetzt werden können. Gegenüber den herkömmlichen Düngemitteln weisen sie einen verminderten Phosphatgehalt auf und sind wegen ihrer verzögerten Phosphatfreisetzung für nahezu alle Anwendungsfälle besonders vorteilhaft. Das Problem der "Überdüngung" kann bei den erfindungsgemäßen Materialien kaum auftreten. Wegen der guten Schmelz- und Gießbarkeit lassen sich ohne größeren Aufwand entsprechend leicht granulierbare Endprodukte herstellen. Die erfindungsgemäßen Materialien können mit üblichen Hilfs- und/oder Trägerstoffen sowie mit bekannten Düngemitteln, insbesondere Stickstoffträgern, kombiniert werden und mittels bekannter Methoden als Staub (Suspension) oder Granulat auf bzw. in den Boden eingebracht werden.

Als Spurenelemente für Düngemittel kommen im erfindungsgemäßen Material folgende Spurennährstoffe in Betracht: Bor, Kupfer, Mangan, Zink, Eisen, Kobalt, Molybdän. Die erfindungsgemäßen Düngemittel eignen sich besonders für intensiv bebaute Böden, da es jenen besonders an Kalium, Phosphor und Magnesium, gelegentlich auch an Schwefel mangelt.

Da es üblich ist, Schwefel als sog. "Kopfdünger" einzusetzen, kommt die im erfindungsgemäßen Material vorliegende Form des Schwefels, die Möglichkeit einer schnellen Freisetzung, besonders der schwefelhaltigen Kaliumkomponente, dem Anwendungsgebrauch entgegen. (Bei Implantation von derartigen Materialien in lebende Organismen ist hingegen, die Natriumkomponente bevorzugt einzusetzen.)

Die Anwendung als Düngemittel konzentriert sich auf Kleingärten, Wein- und Obstanbau, jedoch wäre es ebenso günstig für Ackerbohnen und Roggen, da hier vergleichsweise viel Calcium dem Boden entzogen wird und calciumreiche Düngemittel ebenfalls aus dem erfindungsgemäßen Zusammensetzungsfeld ableitbar sind.

Obwohl vorzugsweise lediglichder Grunddünger vor der Aussaat ausgebracht wird, empfiehlt es sich auch, den erfindungsgemäßen Depotdünger so anzuwenden. Die auszubringenden Mengen lassen sich auf der Basis der jeweiligen Bodenanalyse im Zusammenhang mit den getesteten Löslichkeiten der Materialien ermitteln (vergl. auch die unten folgenden Analysewerte der Schnellmethode zur Bestimmung der Löslichkeit).

Weiterhin wurde gefunden, daß die erfindungsgemäßen Materialien mit schneller Löslichkeit in der eingangs beschriebenen Zusammensetzung einschließlich der vorteilhaften Zusammensetzungsbereiche erfolgreich als Futtermittelzusatz eingesetzt werden können. Gegenüber herkömmiichen Futtermittelzusatz auf Basis von Metaphosphatgläsern werden durch verringerten aber ausreichenden Phosphatgehalt, der langsam im tierischen Körper umgesetzt wird, vorteilhafte Wirkungen erzielt. Insbesondere günstig ist die Form des Subkutan-Implantatkörpers, der zusätzlich noch die Spurenelemente wie Kupfer, Kobalt und/oder Selen in gewünschter Menge enthält, d.h., die Konzentration dieser Bestandteile kann im Implantatkörper durchaus höhere Werte im Prozentbereich einschließen.

Die erfindungsgemäßen Futtermittel können weiterhin übliche Hilfs- und/oder Trägerstoffe sowie bekannte Futtermittel in sinnvollen Beimischungen enthalten. Sie können als Pellets oder in Suspension in einer Flüssignahrung verabreicht werden; vorteilhaft können sie jedoch in der bereits genannten Subkutan- Implantatkörperform und damit gezielt zur Behandlung von Magelerscheinungen in der Tierproduktion verwendet werden. Die Anwendungsmengen können entsprechend den Erfordernissen aus den weiter unten gegebenen Lösungsversuchen grob ermittelt werden. Je höher jedoch der Spurenelementezusatz gewählt wird, um so stärker treten veränderte (verzögerte) Lösungsmechanismen auf, die in entsprechenden Abschätzungen Berücksichtigung finden müssen.

Um einen Vergleich zur In-vitro-Löslichkeit verschiedener Substanzen ziehen zu können, wurden zwei verschiedene Wege beschritten, einmal die Löslichkeit in einer Differenzialkreislaufzelle und zum anderen eine Schnellmethode, die hier zur Kenntnis gegeben werden soll:

Das zu untersuchende Material wird zerkleinert und die für die Bestimmung gewählte Kornfraktion von 315 - 400 $\mu$m wird entnommen. Das Probematerial wird mit Ethanol gewaschen und anschließend bei 110 °C getrocknet. Es werden 10 Proben von jeweils ca. 2g der Untersuchungssubstanz auf der Analysenwaage eingewogen. Bidestilliertes Wasser wird auf 37 °C erhitzt und jeweils 200 ml im Becherglas werden mit den eingewogenen ca. 2g versetzt. Diese Probe bleibt 24 h im Brutschrank bei 37 °C, abgedeckt mit einem Uhrglas, stehen. Nach dieser Zeit werden die Proben in vorher ausgewogene Fritten quantitativ überführt. Danach werden die Filter mit der Probensubstanz bei 110 °C getrocknet. Nach dem Abkühlen im Exikator erfolgt die erneute Wägung zur Ermittlung des Gewichtsverlustes nach:

(Einwaage in mg - Auswaage in mg) * 1000/Einwaage in mg = Ergebnis in mg Substanzverlust/ g Einwaage

Sodann wird die Standardabweichung berechnet.

Nach dieser Methode ergeben sich folgende Werte:

| Material bzw. Material-Code[*)] | | mg Substanzverlust/ g Einwaage |
|---|---|---|
| $Ca_3(PO_4)_2$ | Charge 1<br>Charge 2<br>Charge 3<br>Charge 4 | 3,1 ± 0,39<br>3,0 ± 0,63<br>2,9 ± 0,60<br>2,2 ± 0,16 |
| $4CaO^*P_2O_5$ | Charge 1 | 1,8 ± 0,72 |
| a | Charge 1<br>Charge 2<br>Charge 3 | 5,9 ± 0,27<br>6,6 ± 0,55<br>6,2 ± 0,28 |
| b<br>c | | 5,7 ± 0,84<br>14,7 ± 0,77 |
| d | Charge 1, unbehandelt<br>Charge 2, unbehandelt<br>Charge 1, behandelt | 93,6 ± 3,57<br>87,6 ± 1,22<br>23,1 ± 1,23 |
| e | unbehandelt<br>behandelt | 36,6 ± 1,68<br>3,8 ± 0,77 |
| f<br>g<br>o<br>p | (glasig, abgeschreckt)<br><br>Charge 10,unbehandelt<br>Charge 10,unbehandelt | 54,9 ± 7,12<br>9,8 ± 1,98<br>188,0 ± 0,99 (n = 5)<br>244,9 ± 0,5 (n = 6) |

[*)] Zusammensetzungen siehe Tabelle 1

Nach den Ergebnissen der Schnellmethode zur Bestimmung der Löslichkeit zeichnet es sich ab, daß man eine Grobeinteilung der Materialien nach den Hauptkristallphasen vornehmen kann:
Rhenanit bzw. Mischkristalle des Rhenanits ca. 3...10 mg/g Phase "A" bzw. Mischkristalle der Phase "A" ca. 1....4 mg/g Phase "X" bzw. Mischkristalle der Phase "X" ca. 7...15 mg/g. Unter dem Zusatz der Sulfatanteile bzw. nach Durchführung einer entsprechenden Behandlung (Auslaugung) von Materialien mit hohen Sulfatanteilen, besitzt diese Einteilung dann natürlich keine Gültigkeit mehr; diese Werte werden dann wunschgemäß noch erheblich übertroffen.

Festzustellen bleibt jedoch generell, daß insbesondere Materialien, die die (neue) Phase "X" bzw. Mischristalle der Phase "X" enthalten, sich für die Herstellung der reinen und der mit Sulfatanteilen versehenen Mischschmelzen und daraus gewonnenen Produkten besonders gut eignen. Dies drückt sich u.a. in der guten Gießbarkeit der Schmelzen, der Homogenität der Materialien, der Auslaugfähigkeit bei Anwesenheit von Sulfaten etc. aus.

Ausführungsbeispiele

Die vorangestellten Ausführungen, die lediglich den vollen Umfang der Erfindung erkennen lassen, werden im folgenden durch wenige Beispiele konkreter erläutert. Die in der Tabelle 1 genannten Ansätze wurden geschmolzen und entsprechend der nachfolgenden Anwendungstests zerkleinert. Es wurden die kristallinen Phasen bestimmt,die Löslichkeit nach der beschriebenen Schnellmethode und in der Differentialkreislaufzelle getestet. Ein Teil dieser Ergebnisse wurde bereits im vorangestellten Abschnitt dargestellt.

Im folgenden werden nun Beispiele zu einigen Untersuchungen im Hinblick auf die Anwendung gegeben.

EP 0 541 546 B1

Tabelle 1

| Liste der Zusammensetzungsbeispiele: | | | | | | | |
|---|---|---|---|---|---|---|---|
| Code-Bezeichnung | Oxid-Zusammensetzung in Masse-% | | | | | | |
| | CaO | MgO | $Na_2O$ | $K_2O$ | $P_2O_5$ | $SiO_2$ | Zusätze |
| a | 24,0 | 5,4 | 8,3 | 13,3 | 40,1 | 8,9 | - |
| b | 25,11 | 5,52 | 13,70 | 6,94 | 41,48 | 7,38 | - |
| c | 31,5 | - | 8,3 | 13,3 | 40,1 | 8,9 | - |
| d | 24,0 | 5,4 | 8,3 | 13,3 | 40,1 | 8,9 | 10 $Na_2SO_4$ |
| e | 25,11 | 5,52 | 13,70 | 6,94 | 41,48 | 7,38 | 10 $Na_2SO_4$ |
| f | 26,63 | 6,04 | 9,27 | 14,12 | 34,05 | 9,05 | - |
| g | 18,68 | 6,73 | 10,32 | 15,64 | 38,63 | 10,00 | - |
| h | 25,76 | 4,64 | 11,27 | 17,48 | 40,85 | - | - |
| i | 25,76 | 4,64 | 11,27 | 17,48 | 40,85 | - | 8 $Na_2SO_4$ |
| j | 32,75 | - | 13,7 | 6,94 | 41,48 | 7,38 | - |
| k | 32,75 | - | 13,7 | 6,94 | 41,48 | 7,38 | 4 $Na_2SO_4$ |
| l | 24,35 | 5,52 | 8,84 | 13,54 | 40,54 | 7,21 | - |
| m | 24,35 | 5,52 | 8,84 | 13,54 | 40,54 | 7,21 | 4 $K_2SO_4$ |
| n | 24,35 | 5,52 | 8,84 | 13,54 | 40,54 | 7,21 | 10 $K_2SO_4$ |
| o | 24,35 | 5,52 | 8,84 | 13,54 | 40,54 | 7,21 | 30 $Na_2SO_4$ |
| p | 24,0 | 5,4 | 8,3 | 13,3 | 40,1 | 8,9 | 20 $Na_2SO_4$ |
| q | 24,0 | 5,4 | 8,3 | 13,3 | 40,1 | 8,9 | 10 $Na_2SO_4$ und 10 $K_2SO_4$ |
| r | 24,35 | 5,52 | 8,84 | 13,54 | 40,54 | 7,21 | 4,5 $Na_2SO_4$ und 4,5 $K_2SO_4$ |
| s | 25,76 | 4,64 | 11,27 | 17,48 | 40,85 | - | 3 $Na_2SO_4$ und 18 $K_2SO_4$ |

Beispiel 1

Ein Material der Zusammensetzung g wurde nach dem Schmelzen und Abkühlen unter normalen Bedingungen zerkleinert. Eine Kornfraktion von 200 bis 500 $\mu$m wurde ausgewählt für die nachfolgenden Tierversuche.

Das Material wurde sowohl subkutan als auch in den Knochen von Ratten und Kaninchen implantiert. Nach 4, 8 und 12 Wochen Liegezeit war das Material in beiden Fällen noch nachweisbar, obwohl der Mengenanteil bzw. die Korngröße beträchtlich abgenommen hatten. Im Knochengewebe wurde das Material direkt von Knochengewebe kontaktiert, während bei der Subkutanimplantation eine feste bindegewebige Einscheidung impliziert worden war.

Beispiel 2

Ein Material der Zusammensetzung f wurde geschmolzen, zu einer Platte vergossen und nach dem langsamen Abkühlen im Ofen zerkleinert. Das Granulat der Kornfraktion von 200 bis 500 $\mu$m wurde analog zum Beispiel 1 implantiert.

Die histologischen Untersuchungen ergaben, daß das Material - wie alle implantierten Materialien überhaupt - entzündungslos einheilte. In diesem Falle zeigte sich ferner, daß das Material im Knochengewebe nach 12 bzw. 20 Wochen vollständig resorbiert war. Hingegen konnten Partikelreste im Bindegewebe nach Subkutanimplantation nachgewiesen werden.

Beispiel 3

Analog zu Beispiel 2 wurde Material der Zusammensetzung a hergestellt und implantiert. Während im Knochen noch Reste des implantierten Granulats nach 15 Wochen nachweisbar waren, war es im Subkutangewebe nach dieser Zeit bereits vollständig resorbiert worden.

9

Beispiel 4

Ein Material der Zusammensetzung d wurde bei 1500 °C geschmolzen, zu einer Platte vergossen, die anschließend nach dem Abkühlen zerkleinert wurde. Das daraus hergestellte Granulat der Kornfraktion von 200 - 500 $\mu$m wurde einmal vorbehandelt, d.h., bei 90 °C 30 min lang in dest. Wasser gekocht, und zum anderen unbehandelt in Versuchsschweine in den Knochen implantiert.

Nach 20 Wochen Liegedauer waren beide Materialien vollständig resorbiert. Es wurde jedoch keine Knochenbildung an diesen Stellen impliziert, wodurch sich das Material zur Auffüllung von Hohlräumen eignet, bei denen lediglich Bindegewebe induziert werden soll.

Beispiel 5

Die Materialherstellung und die tierexperimentelle Testung erfolgte exakt nach Beispiel 4.

In beiden Fällen wurde jedoch 55 Masse-% des erfindungsgemäßen Materials mit 45 Masse-% eines oberflächenmodifizierten Tricalciumphosphat(TCP)-Granulats (entsprechend WP-DD 258713 A 3) vermischt und implantiert.

In diesen Fällen zeigte sich eine Knochenbildung in den künstlich gesetzten Defekten, wobei noch Reste des TCP nach diesem Zeitraum von 20 Wochen noch nachweisbar waren. Allerdings sind diese Implantatreste vergleichsweise extrem gering als im Falle der Auffüllung der Knochendefekte mit reinem oberflächenmodifizierten TCP.

Durch diese kombinierte Anwendung ist der Knochen nach einer entsprechenden Einheilphase voll funktionsfähig.

**Patentansprüche**

1. Glasiges oder glasig-kristallines Material mit schneller Löslichkeit, dadurch gekennzeichnet, daß das Gemenge vor dem Brennen des Materials folgende chemische Zusammensetzung (in Masseteile in % und auf Oxidbasis berechnet) aufweist
20 bis 55 % $CaO$, 5 bis 25 % $Na_2O$, O bis 15 % $K_2O$ O bis 15 % $MgO$, 30 bis 50 % $P_2O_5$, 0 bis 15 % $SiO_2$ 0 bis 40 % $Na_2SO_4$ und/oder $K_2SO_4$
und daß das glasig-kristalline Material als kristalline Hauptbestandteile mindestens eine der Phasen des Rhenanits, Mischkristalle des Rhenanits, die Phase "A", Mischkristalle der Phase "A", die Phase "X" und/oder Mischkristalle der Phase "X" enthält, wobei die Phase "X" eine Phase ist, die der Phase "A" ähnelt, sich aber durch erhebliche Linienverschiebungen und Intensitätsänderungen sowie durch das Fehlen einer starken Beugungslinie an der Netzebene (421) röntgendiffraktometrisch unterscheidet.

2. Material nach Anspruch 1, dadurch gekennzeichnet, daß es besteht aus
20-55 % $CaO$, vorzugsweise 21-50, insbesondere 23-50 5-25 % $Na_2O$, vorzugsweise 5-20, insbesondere 6-20 0,1-15 % $K_2O$, vorzugsweise 0,1-14, insbesondere 2-14 0,1-15 % $MgO$, vorzugsweise 0,1-12, insbesondere 0,5-10 30-50 % $P_2O_5$, vorzugsweise 32-48, insbesondere 35-48 0,1-15 % $SiO_2$, vorzugsweise 2-15, insbesondere 3-15 0,1-35 % $Na_2SO_4$ und/oder $K_2SO_4$, vorzugsweise 0,1-20, insbesondere 0,1-15.

3. Material nach Anspruch 1 oder 2, dadurch gekennzeichnet, daß es zusätzlich Glaserit und/oder kristallines Kaliumsulfat enthält.

4. Material nach einem der Ansprüche 1 bis 3, dadurch gekennzeichnet, daß als kristalline Hauptbestandteile Rhenanit bzw. Mischkristalle des Rhenanits vorliegen, die folgende chemische Zusammensetzung aufweisen (in Masseanteile in % und auf Oxidbasis berechnet):
30 - 40 % $CaO$; 15 - 20 % $Na_2O$; 0 - 1 % $K_2O$, vorzugsweise 0,01 - 0,1 %; 0 - 5 % $MgO$, vorzugsweise 0,1 - 5 %, 40 - 55 % $P_2O_5$; 0 - 15 % $SiO_2$, vorzugsweise 0,1 - 8 %; 0 - 30 % $Na_2SO_4$ und/oder $K_2SO_4$, vorzugsweise 0,1 - 25.

5. Material nach einem der Ansprüche 1 bis 3, dadurch gekennzeichnet, daß als kristalline Hauptbestandteile die Phase "A" bzw. Mischkristalle der Phase "A" vorliegen, die folgende chemische Zusammensetzung aufweisen:
40 - 50 % $CaO$; 8 - 20 % $Na_2O$; 0 - 1 % $K_2O$, vorzugsweise 0,1 - 1 % 0 - 5 % $MgO$, vorzugsweise 0,1 - 5 %; 40 - 50 % $P_2O_5$; 3 - 20 % $SiO_2$; 0 - 30 % $Na_2SO_4$ und/oder $K_2SO_4$, vorzugsweise 0,1 bis 25 %.

6. Material nach einem der Ansprüche 1 bis 3, dadurch gekennzeichnet, daß als kristalline Hauptbestandteile die Phasen "X" bzw. Mischkristalle der Phase "X" vorliegen, die folgende chemische Zusammensetzung aufweisen:
22 - 45 % CaO; 8 - 20 % $Na_2O$; 0 - 14 % $K_2O$, vorzugsweise 0,1 - 14 %; 0 - 15 % MgO, vorzugsweise 0,1 - 15 %; 30 bis 55 % $P_2O_5$; 0 - 15 % $SiO_2$, vorzugsweise 0,1 bis 15 %; 0 - 40 % $Na_2SO_4$ und/oder $K_2SO_4$, vorzugsweise 0,1 - 35 %.

7. Material nach einem der Ansprüche 1 bis 6, dadurch gekennzeichnet, daß es einen Arzneimittelwirkstoff trägt und/oder in einer Trägerfolie vorliegt.

8. Verfahren zur Herstellung eines glasigen oder glasigkristallinen Materials mit schneller Löslichkeit, dadurch gekennzeichnet, daß ein Gemenge, bestehend aus (in Masseanteilen in % und auf Oxidbasis berechnet)
20 bis 55 % CaO; 5 bis 25 % $Na_2O$; 0 bis 15 % $K_2O$; 0 bis 15 % MgO; 30 bis 50 % $P_2O_5$; 0 bis 15 % $SiO_2$; 0 bis 40 % $Na_2SO_4$ und/oder $K_2SO_4$
mindestens 10 Minuten bei einer Temperatur von 1200 bis 1580 °C gesintert oder geschmolzen wird und a) das gesinterte Material abgekühlt wird, oder b) die Schmelze entweder mit einer Abkühlgeschwindigkeit von größer etwa 5 . $10^2$ °C pro Minute in den glasigen Zustand bei Raumtemperatur überführt wird, oder bei Abkühlung unter normaler Abkühlgeschwindigkeit oder unter einer Abkühlgeschwindigkeit, die langsamer als etwa 35 °C pro Minute erfolgt, spontan kristallisiert wird; und gegebenenfalls c) das spontan kristallisierte Material einem üblichen Temperungsprozeß unterzogen wird im Temperaturbereich von ca. 600 bis 1200 °C in Abhängigkeit von der chemischen Zusammensetzung, um den kristallinen Anteil noch weiter zu erhohen, wobei die Kristallphasen des Rhenanits, Mischkristalle des Rhenanits, der Phase "A", Mischkristalle der Phase "A" der Phase "X" Mischkristalle der Phase "X", Glaserit und/oder kristallines Kaliumsulfat in Erscheinung treten, in der Regel jedoch der Anteil der Hauptkristallphase weiter erhöht wird, wobei die Phase "X" eine Phase ist, die der Phase "A" ähnelt, sich aber durch erhebliche Linienverschiebungen und Intensitätsänderungen sowie durch das Fehlen einer starken Beugungslinie an der Netzebene (421) röntgendiffraktometrisch unterscheidet.

9. Verwendung des Materials nach einem der Ansprüche 1 bis 8, dadurch gekennzeichnet, daß man es zur Herstellung eines resorbierbaren Knochenersatzmaterials oder eines Materials zur Bindegewebsinduktion einsetzt.

10. Verwendung des Materials nach einem der Ansprüche 1 bis 8, dadurch gekennzeichnet, daß man es als Beschichtungsmaterial für Implantatformkörper für den Hartgewebeersatz aus Metallen oder Legierungen und/oder Keramiken, vorzugsweise $Al_2O_3$-Keramiken, einsetzt.

**Claims**

1. Vitreous or vitreous-crystalline, rapidly dissolving material, characterized in that it presents the following chemical composition (calculated in wt.% and on oxide basis) before combustion:
20 - 55 % CaO, 5 - 25 % $Na_2O$, 0 - 15 % $K_2O$ 0 - 15 % MgO, 30 - 50 % $P_2O_5$, 0 - 15 % $SiO_2$ 0 - 40 % $Na_2SO_4$ and/or $K_2SO_4$
and characterized in that the vitreous-crystalline material contains as crystalline main components at least one of the rhenanite phases, mixed crystals of the rhenanite, "A" phase, mixed crystals of the "A" phase, "X" phase and/or mixed crystals of the "X" phase, with the "X" phase being a phase which resembles the "A" phase but differs X-ray diffractometrically by strong line shifts and blackening variations as well as by the absence of a strong diffraction line at the lattice plane (421).

2. Material according to claim 1, characterized in that it consists of
20 - 55 % CaO, preferably 21 - 50, in particular 23 - 50 5 - 25 % $Na_2O$, preferably 5 - 20, in particular 6 - 20 0.1 - 15 % $K_2O$, preferably 0.1 - 14, in particular 2 - 14 0.1 - 15 % MgO, preferably 0.1 - 12, in particular 0.5 - 10 30 - 50 % $P_2O_5$, preferably 31 - 48, in particular 35 - 48 0.1 - 15 % $SiO_2$, preferably 2 - 15, in particular 3 - 15 0.1 - 35 % $Na_2SO_4$ and/or $K_2SO_4$, preferably 0.1 - 20, in particular 0.1 - 15.

3. Material according to either claim 1 or 2, characterized in that it contains in addition glaserite and/or crystalline potassium sulphate.

4. Material according to one of claims 1 to 3, characterized in that it has as crystalline main components rhenanite or also its mixed crystals presenting the following chemical composition (calculated in wt.% and on oxide basis):

30 - 40 % CaO; 15 - 20 % $Na_2O$; 0 - 1 % $K_2O$, preferably 0.01 - 0.1 %; 0 - 5 % MgO, preferably 0.1 - 5 %, 40 - 55 % $P_2O_5$; 0 - 15 % $SiO_2$, preferably 0.1 - 8 %; 0 - 30 % $Na_2SO_4$ and/or $K_2SO_4$, preferably 0.1 - 25.

5. Material according to one of claims 1 to 3, characterized in that there are as crystalline main components the "A" phase or also its mixed crystals presenting the following chemical composition:

40 - 50 % CaO; 8 - 20 % $Na_2O$; 0 - 1 % $K_2O$, preferably 0.1 - 1 %; 0 - 5 % MgO, preferably 0.1 - 5 %; 40 - 50 % $P_2O_5$; 3 - 20 % $SiO_2$; 0 - 30 % $Na_2SO_4$ and/or $K_2SO_4$, preferably 0.1 - 25 %.

6. Material according to one of claims 1 to 3, characterized in that there are as crystalline main components the "X" phases or also their mixed crystals presenting the following chemical composition:

22 - 45 % CaO; 8 - 20 % $Na_2O$; 0 - 14 % $K_2O$, preferably 0.1 - 14 %; 0 - 15 % MgO, preferably 0.1 - 15 %; 30 - 55 % $P_2O_5$; 0 - 15 % $SiO_2$, preferably 0.1 - 15 %; 0 - 40 % $Na_2SO_4$ and/or $K_2SO_4$, preferably 0.1 - 35 %.

7. Material according to one of claims 1 to 6, characterized in that it carries a medicinal active substance and/or is present in a carrier foil.

8. Process for the production of a vitreous or vitreous-crystalline rapidly dissolving material, characterized in that a batch consisting of (calculated in wt.% and on oxide basis)

20 - 55 % CaO; 5 - 25 % $Na_2O$; 0 - 15 % $K_2O$; 0 - 15 % MgO; 30 - 50 % $P_2O_5$; 0 - 15 % $SiO_2$; 0 - 40 % $Na_2SO_4$ and/or $K_2SO_4$ is sintered or melted for at least 10 minutes at a temperature ranging from 1200 to 1580 °C and a) the sintered material is cooled or b) the melt is either transformed into the vitreous state at room temperature at a cooling rate above about $5 . 10^2$ °C per minute or spontaneously brought into a crystallized form either by cooling at normal cooling rate or at a cooling rate slower than about 35 °C per minute, and that, if need be, c) the spontaneously crystallized material is subjected to a usual annealing process in the temperature range from about 600 to 1200 °C according to the chemical composition in order to further increase the crystalline portion, with the crystalline phases of the rhenanite, mixed crystals of the rhenanite, of the "A" phase, mixed crystals of the "A" phase, of the "X" phase, mixed crystals of the "X" phase, glaserite and/or crystalline potassium sulphate showing up, in general, however, the portion of the main crystalline phasis is further increased, with the "X" phase being a phase which resembles the "A" phase but differs X-ray diffractometrically by strong line shifts and blackening variations as well as by the absence of a strong diffraction line at the lattice plane (421).

9. The use of the material according to one of claims 1 to 8, characterized in that it is used as coating material for implant bodies destined for the hard tissue replacement consisting of metals or alloys and/or ceramics, preferably $Al_2O_3$ ceramics.

10. The use of the material according to one of claims 1 to 8, characterized in that it is used for the production of an absorbable bone replacement material or of a material for the connective tissue induction.

**Revendications**

1. Matière vitreuse ou vitreuse-cristalline rapidement soluble, caractérisée en ce que le mélange présente la composition chimique suivante avant la combustion de la matière (calculée en % en poids et à la base d'oxyde):

20 - 55 % CaO, 5 - 25 % $Na_2O$, 0 - 15 % $K_2O$ 0 - 15 % MgO, 30 - 50 % $P_2O_5$, 0 - 15 % $SiO_2$ 0 - 40 % $Na_2SO_4$ et/ou $K_2SO_4$

et en ce que la matière vitreuse-cristalline contient comme composants principaux cristallins une des phases du rhénanite, des cristaux mixtes du rhénanite la phase "A" des cristaux mixtes de la phase "A" la phase "X" et/ou des cristaux mixtes de la phase "X" la phase "X" étant une phase qui ressemble à la phase "A" mais qui se distingue diffractométriquement par d'importants décalages des raies et changements d'intensité ainsi que par l'absence d'une forte ligne de diffraction sur le plan réticulaire (421).

2. Matière selon la revendication 1, caractérisée en ce qu'elle consiste de
20 - 55 % CaO, de préférence 21 - 50, en particulier 23 - 50, 5 - 25 % $Na_2O$, de préférence 5 - 20, en particulier 6 - 20, 0,1 - 15 % $K_2O$, de préférence 0,1 - 14, en particulier 2 - 14, 0,1 - 15 % MgO, de préférence 0,1 - 12, en particulier 0,5 - 10, 30 - 50 % $P_2O_5$, de préférence 32 - 48, en particulier 35 - 48, 0,1 - 15 % $SiO_2$, de préférence 2 - 15, en particulier 3 - 15, 0,1 - 35 % $Na_2SO_4$ et/ou $K_2SO_4$, de préférence 0,1 - 20, en particulier 0,1 - 15.

3. Matière selon la revendication 1 ou 2, caractérisée en ce qu'elle contient en plus du glasérite et/ou du sulfate de potassium cristallin.

4. Matière selon une des revendications 1 à 3, caractérisée en ce qu'il y a comme composants principaux cristallins du rhénanite ou bien des cristaux mixtes du rhénanite présentant la composition chimique suivante (calculée en % en poids et à la base d'oxyde):
30 - 40 % CaO; 15 - 20 % $Na_2O$; 0 - 1 % $K_2O$, de préférence 0,01 - 0,1 %; 0 - 5 % MgO, de préférence 0,1 - 5 %, 40 - 55 % $P_2O_5$; 0 - 15 % $SiO_2$, de préférence 0,1 - 8 %; 0 - 30 % $Na_2SO_4$ et/ou $K_2SO_4$, de préférence 0,1 - 25.

5. Matière selon une des revendications 1 à 3, caractérisée en ce qu'il y a comme composants principaux cristallins la phase "A" ou bien des cristaux mixtes de la phase "A" présentant la composition chimique suivante:
40 - 50 % CaO; 8 - 20 % $Na_2O$; 0 - 1 % $K_2O$, de préférence o,1 - 1 %; 0 - 5 % MgO, de préférence 0,1 - 5 %; 40 - 50 % $P_2O_5$; 3 - 20 % $SiO_2$; 0 - 30 % $Na_2SO_4$ et/ou $K_2SO_4$, de préférence 0,1 - 25 %.

6. Matière selon une des revendications 1 à 3, caractérisée en ce qu'il y a comme composants principaux cristallins les phases "X" ou bien des cristaux mixtes de la phase "X" présentant la composition chimique suivante:
22 - 45 % CaO; 8 - 20 % $Na_2O$; 0 - 14 % $K_2O$, de préférence o,1 - 14 %; 0 - 15 % MgO, de préférence O,1 - 15 %; 30 - 55 % $P_2O_5$; 0 - 15 % $SiO_2$, de préférence 0,1 - 15 %; 0 - 40 % $Na_2SO_4$ et/ou $K_2SO_4$, de préférence 0,1 - 35 %.

7. Matière selon une des revendications 1 à 6, caractérisée en ce qu'elle porte un agent médicamenteux et/ou est présente dans une feuille porteuse.

8. Procédé de la fabrication d'une matière vitreuse ou vitreuse-cristalline rapidement soluble, caractérisé en ce qu'un mélange consistant de (calculé en % en poids et à la base d'oxyde):
20 - 55 % CaO; 5 - 25 % $Na_2O$; 0 - 15 % $K_2O$; 0 - 15 % MgO; 30 - 50 % $P_2O_5$; 0 - 15 % $SiO_2$; 0 - 40 % $Na_2SO_4$ et/ou $K_2SO_4$
est fritté ou fondu pour au moins 10 minutes à une température de 1200 à 1580 °C et que a) la matière frittée est fait refroidir ou b) la fonte est transformée en état vitreux à température ambiante soit à une vitesse de refroidissement supérieure à environ 5 . $10^2$ °C par minute soit est cristallisée spontanément sous refroidissement à une vitesse de refroidissement normale ou à une vitesse de refroidissement inférieure à environ 35 °C par minute, et que c) la matière spontanément cristallisée est, le cas échéant, assujettie à un processus de recuit usuel dans la plage de températures allant d'environ 600 jusqu'à 1200 °C en fonction de la composition chimique afin d'augmenter encore plus la portion cristalline, les phases cristallines du rhénanite, des cristaux mixtes du rhénanite, de la phase "A", des cristaux mixtes de la phase "A", de la phase "X", des cristaux mixtes de la phase "X", du glasérite et/ou du sulfate de potassium cristallin se montrant, mais en règle générale la portion de la phase cristalline principale étant augmentée encore plus et la phase "X" étant une phase qui ressemble à la phase "A" mais qui se distingue diffractométriquement par d'importants décalages des raies et changements d'intensité ainsi que par l'absence d'une forte ligne de diffraction sur le plan réticulaire (421).

9. Emploi de la matière selon une des revendications 1 à 8, caractérisé en ce qu'on l'utilise pour la fabrication d'une matière substituante absorbable des os ou d'une matière pour l'induction du tissu conjonctif.

**10.** Emploi de la matière selon une des revendications 1 à 8, caractérisé en ce qu'on l'utilise comme matière d'enduction pour des corps profilés d'implant pour la substitution du tissu dur par des substituts en métal ou des alliages et/ou des céramiques, de préférence des céramiques en $Al_2O_3$.